# EUROPEAN PATENT SPECIFICATION

(11) **EP 2 413 839 B1**
(45) Date of publication and mention of the grant of the patent: **07.05.2014**
(21) Application number: 10713073.4
(22) Date of filing: 01.04.2010
(51) Int. Cl.: A61F 2/94, A61F 2/04

(54) **SYSTEM AND METHOD FOR MAINTAINING PATENCY OF A STENT USING A MAGNET**
SYSTEM UND VERFAHREN ZUR AUFRECHTERHALTUNG DER DURCHGÄNGIGKEIT EINES STENTS MIT EINEM MAGNETEN
SYSTÈME ET PROCÉDÉ DE MAINTIEN DE LA PERMÉABILITÉ D'UN STENT À L'AIDE D'UN AIMANT

(30) Priority: 02.04.2009 US 166109 P
(43) Date of publication of application: 08.02.2012
(73) Proprietor: Cook Medical Technologies LLC, Bloomington, IN 47404 (US)
(72) Inventor: SKERVEN, Gregory, J., Kernersville NC 27284 (US)
(74) Representative: Garratt, Peter Douglas
(86) International application number: PCT/US2010/029561
(87) International publication number: WO 2010/114962

(56) References cited:
- WO-A2-2005/072169
- WO-A2-2007/127419
- WO-A2-2007/149404
- US-A- 5 004 454
- US-A- 5 730 732
- US-A1- 2008 269 546

## Description

### RELATED APPLICATIONS

The present patent document claims the benefit of the filing date of Provisional U.S. Patent Application Serial No. 61/166,109, filed April 2, 2009.

### FIELD OF THE INVENTION

The present invention relates to stents, including plastic tubular stents adapted for use in the biliary tract.

### BACKGROUND OF THE INVENTION

Stents are frequently used to enlarge, dilate, or maintain the patency of narrowed body lumens. Non-expandable tubular stents are typically made from plastic and contain a lumen extending throughout.

Implantation of biliary stent structures provides treatment for various conditions, such as obstructive jaundice. Biliary stenting treatment approaches can be used to provide short-term treatment of conditions such as biliary fistulae or giant common duct stones. Biliary stents may be implanted to treat chronic conditions such as postoperative biliary stricture, primary sclerosing cholangitis, and chronic pancreatitis.

A biliary stent can be made in the form of a polymer tube that can be advanced on a delivery catheter through an endoscope and into the bile duct where it is deployed. The tubular stent is selected to be sufficiently strong to resist collapse to maintain an open lumen through which digestive liquids can flow into the digestive tract. Among the desirable features of such a stent is that it be longitudinally flexible to be advanced along a path that may include sharp bends. It is also desirable that the stent maintain its intended position within the bile duct without migrating from that position.

As bodily fluid travels through the lumen of the stent, cumulative matter within the bodily fluid adheres to the inner surface of the stent. Cumulative matter is material traversing the stent that if undisturbed, would otherwise accumulate on the passageway surfaces to reduce the diameter of the flow path there through, reduce the stent's patency, and could eventually clog the stent. Cumulative matter includes, but is not limited to biofilm, bacterial growth, and sludge deposition. A biliary stent can become occluded within a bile duct, as cumulative material, such as an encrustation of amorphous biological material and bacteria, and/or sludge, accumulates on the surface of the stent gradually obstructing the lumen of the stent. Biliary sludge is an amorphous substance often containing crystals of calcium bilirubinate and calcium palimitate, along with significant quantities of various proteins and bacteria. Sludge can deposit rapidly upon implantation in the presence of bacteria. For example, bacteria can adhere to plastic stent surfaces through pili or through production of a mucopolysaccharide coating. Bacterial adhesion to the surface of a stent lumen surface can lead to occlusion of the stent lumen as the bacteria multiply within a glycocalyx matrix of the sludge to form a biofilm over the sludge within the lumen of an implanted drainage stent. The biofilm can provide a physical barrier protecting encased bacteria from antibiotics. With time, an implanted biliary stent lumen can become blocked, thereby restricting or blocking bile flow through the biliary stent. As a result, a patient can develop symptoms of recurrent biliary obstruction due to restricted or blocked bile flow through an implanted biliary stent, which can be complicated by cholangitis and sepsis.

Often such conditions are treated by antibiotics and/or endoscopic replacement of an obstructed biliary stent. Typically, biliary stents need replacing every three months. Replacement procedures cause medical risk and financial strain to the patient.

Reference is directed to WO 2007/127419 which discloses a system for counteracting the deposition of material on devices that dwell in the urinary tract and which includes an operably connected motion-inducing actuator. In one embodiment, an actuator and an energy receiver both take the form of a series of concentric rings. Movement, involving expansion and contraction of the device radially at the site of the actuators has the effect of dislodging and preventing adherence of particles to the device. Reference is also directed to WO 2007/149404 which discloses a stent including a mass moveably disposed therein, wherein movement of the stent tends to dislodge any cumulative matter accumulated whithin the stent. The mass may comprise a weighted object free to move about the stent lumen and formed of ceramic, stainless steel or gold.

There exists a need in the art for an implantable medical device that prevents or reduces the biofilm and sludge deposition process inside the lumen of implantable drainage stents, such as biliary stents, at little cost and with minimal patient and medical personnel intervention. There is a need for a stent that resists clogging by using a non-invasive, mechanical means that does not require the use of electricity or expensive equipment.

### BRIEF SUMMARY OF THE INVENTION

The scope of the present invention is set forth in the appended claims. There is disclosed a system for maintaining the patency of a plastic tubular stent is provided. The system includes a plastic tubular stent having a lumen and one or more magnetically reactive objects; wherein the one or more magnetically reactive objects are movably disposed within the lumen; and an actuation device, separate from the plastic tubular stent.

The lumen is configured to retain the magnetically reactive object within the plastic tubular stent; and the magnetically reactive object of the plastic tubular stent is configured to at least partially dislodge cumulative matter, deposited within the plastic tubular stent, when the actuation device is passed near the plastic tubular stent.

### BRIEF DESCRIPTION OF THE SEVERAL VIEWS OF THE DRAWINGS

The embodiments will be further described in connection with the attached drawing figures. It is intended that the drawings included as a part of this specification be illustrative of the embodiments and should in no way be considered as a limitation on the scope of the invention.
**Fig. 1A** is a partial cross sectional perspective view of a stent having a magnetically reactive object for maintaining the patency of the stent;
Fig. **1B** is a front view of an actuation device for use with a stent having one or more magnetically reactive objects therein, such as those depicted in Figs. **1A****,** 7, and 8;
**Fig.** 2 is a method for using the system depicted in Figs. **1A****-B;**
**Fig.** 3 is a front view of an actuation device for use with a stent having one or more magnetically reactive objects therein;
**Fig. 4** is a partial cross sectional perspective view of a magnetically reactive object for use in a stent;
**Fig. 5** is a perspective view of a magnetically reactive object for use in a stent;
**Fig. 6** is a perspective view of a magnetically reactive object for use in a stent;
**Fig. 7** is a partial cross sectional perspective view of a stent having multiple magnetically reactive objects for maintaining the patency of the stent; and
**Fig. 8** is a partial cross sectional perspective view of a stent having multiple magnetically reactive objects for maintaining the patency of the stent.

### DETAILED DESCRIPTION OF PRESENTLY PREFERRED EMBODIMENTS

The exemplary embodiments disclosed herein provide a system and gives an example of a method for maintaining the patency of a stent, such as a plastic tubular biliary stent, using a magnet and a magnetically reactive object to at least partially inhibit long-term adherence of cumulative matter to the interior of the stent so that the amount of time the stent can reside within a patient before needing to be replaced is extended The present invention is not limited to any particular type of stent as long as said stent falls under the scope of the appended claims, it is contemplated that stents dwelling in locations other than the biliary can also benefit from the inventive concepts disclosed herein. Furthermore, the present invention is not limited for use within any particular part of the body or for use with humans.

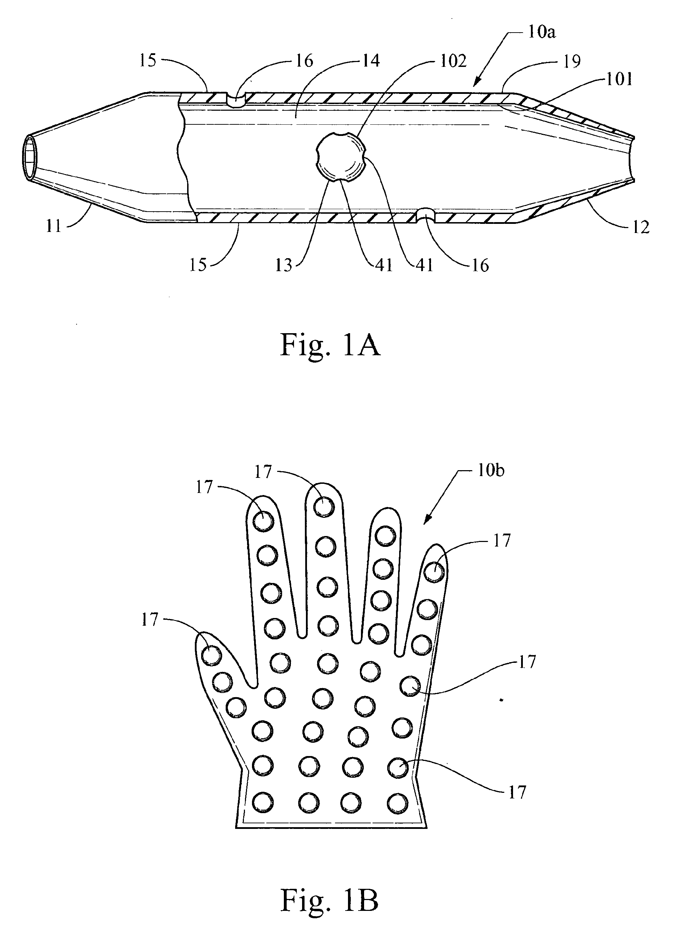

A more detailed description of the embodiments will now be given with reference to **Figs. 1-8****.** Throughout the disclosure, like reference numerals and letters refer to like elements. The present invention is not limited to the embodiments illustrated; to the contrary, the present invention specifically contemplates other embodiments not illustrated but intended to be included in the claims.

**Fig. 1A** is a partial cross sectional perspective view of a stent having a magnetically reactive object for maintaining the patency of the stent. Stent **10a** comprises a first portion **11,** second portion **12,** and a lumen **14** extending throughout. Walls **15** of stent **10a** are about .020 inches thick and the outer diameter of stent **10a** is about 3-10 French. However, other sizes are contemplated depending on the needs of the patient, the size of magnetically reactive object **13,** and the diameter of the body lumen in which stent **10a** will dwell. Side drainage ports/lumens **16** contained within walls **15** allow for additional fluid to pass therethrough. Side drainage ports **16** can be configured in such a way that side drainage lumen **16** do not come in contact with tissue. For example, side drainage lumens **16** can be placed near second portion **12** and/or first **portion 11** of walls **15,** wherein such portion of walls **15** would not come in contact with any portion of the bodily tissue wherein stent **10a** dwells. For example, second portion **12** configured with side drainage lumens **16** can be extended out into the duodenum such that side drainage lumens **16** do not contact and therefore, are not blocked by, any tissue.

Contained within stent **10a** is one or more magnetically reactive objects **13** that are free to move about lumen **14** and will likely inherently move about lumen **14** as the patient moves, although such inherent movement is not required. First portion **11** and second portion **12** of stent **10a** are tapered to act as a magnetically reactive object securing mechanisms to maintain the one or more magnetically reactive objects **13** therein. Magnetically reactive object **13** should be sufficiently sized and shaped such that it does not completely obstruct fluid flow and such that it is able to move about lumen **14** without causing stent **10a** to become dislodged from its dwelling place. The shape of magnetically reactive object **13** is not limited to having a spherical-shape; other shapes are contemplated including, but not limited to, a cube, pyramid, and box. Additionally, magnetically reactive object **13** may have additional materials or coatings attached thereto to aid in the removal of cumulative matter. Magnetically reactive object **13** may further comprise lumen **41** that permits fluid to pass there through.

Using actuation device **10b** which is separate from stent **10a** and located outside of patient, one actuates magnetically reactive object **13** within stent **10a** by manually manipulating magnetically reactive object **13** using external forces that repel or attract magnetically reactive object **13.** It is contemplated that magnetically reactive object and/or any portion of actuation device may be made from a magnetic material (such as a rare earth magnet) or a metallic material that is attracted to or repelled by a magnetic material. Additionally, it is contemplated that if both made from a magnetic material, actuation device and magnetically reactive object may have a polarity that is the same or different such that they will relatively attract or repel each other.

Actuation device **10b** depicted in **Fig. 1B** as a glove further comprising one or more magnets **17**, that may be rare earth magnets, that attract magnetically reactive object **13.**

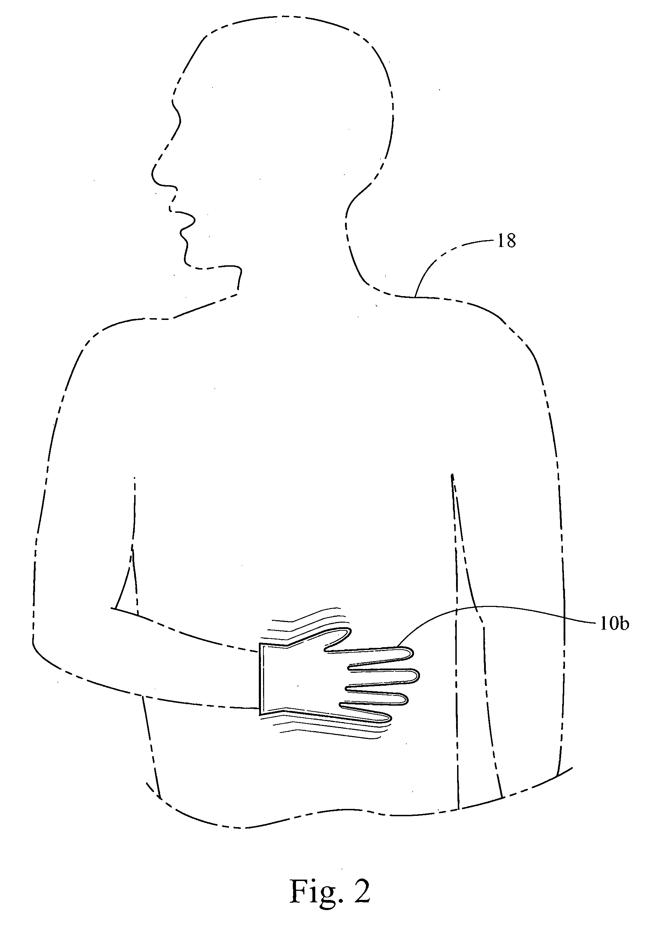

As depicted in **Fig. 2****,** patient **18** (or any other person or thing, including but not limited to, a medical professional), positions actuation device **10b** near stent **10a** and manually manipulates magnetically reactive object **13** by moving/waiving glove **10b** near stent **10a** causing magnetically reactive object **13** to move within lumen 14 of stent **10a** striking walls **15** so as to dislodge cumulative matter attached thereto. The cumulative matter then exits through first portion **11** or second portion **12** of stent **10a.** Cumulative matter can also exit via side drainage lumens **16.** Magnet **17** can be attached to glove **10b** using a variety of techniques, including but not limited to, gluing them in place and/or sewing them in place.

As is readily apparent, patient **18** may be able to perform the method for removing cumulative matter attached to the stent by using actuation device **10b,** such as a glove, without the costly assistance of a medical professional or expensive equipment. Because no electronics or invasive equipment are used, the risk to patient health is minimized. Additionally, because actuation caused by manual manipulation of magnetically reactive object is used, rather than gravity, patients who live a sedentary lifestyle, are bed-bound, or who are otherwise less mobile, are able to benefit from that which is disclosed herein. It is contemplated that patient **18** (or anyone else) can perform the cleaning procedure one, two, or more times daily to help maintain the patency of the stent for a period longer than what a typical stent (such as a plastic biliary stent) would experience.

Although glove **10b** is depicted with a plurality of magnets **17** on the fingers and palm, glove **10b** is not limited to having a plurality of magnets. Rather, it is contemplated that glove **10b** may only have one magnet.

Additionally, although actuation device is depicted as a glove, the actuation device is not limited to a glove. Rather, it further contemplates any handheld actuation device such that magnetically reactive objects are attracted to or repelled from actuation device, including but not limited to, a cloth member, mitt, mitten, wand, strap, housing, or any other device that may be temporarily attached and/or disposed at least partially about a hand, including a magnet alone. Additionally, it is contemplated that actuation device may have one or more magnets or metallic material that may be of differing sizes, dimensions, strengths, and polarities. Likewise, different actuation devices may have one or more magnets or metallic material having different sizes, dimensions, strengths, and polarities, such that if one actuation device does not achieve the desired results, a different actuation device may be used.

Walls **15** may be formed from any suitable biocompatible and biostable material. Walls **15** are preferably resiliently compliant enough to readily conform to the curvature of the duct in which it is to be placed, while having sufficient "hoop" strength to retain its form within the duct. Walls **15** are preferably made from a medium density biocompatible polyethylene, although other materials are contemplated, including but not limited to polyurethane, polytetrafluoroethylene (PTFE), stainless steel, and Nitinol. In one aspect, walls **15** are formed from a polyolefin such as a metallocene catalyzed polyethylene, polypropylene, polybutylene or copolymers thereof. Other suitable materials for walls **15** include polyurethane (such as a material commercially available from Dow Corning under the tradename PELLETHANE); a silicone rubber (such as a material commercially available from Dow Coming under the tradename SILASTIC); a polyetheretherketone (such as a material commercially available from Victrex under the tradename PEEK); vinyl aromatic polymers such as polystyrene; vinyl aromatic copolymers such as styrene-isobutylene copolymers and butadiene-styrene copolymers; ethylenic copolymers such as ethylene vinyl acetate (EVA), ethylene- methacrylic acid and ethylene- acrylic acid copolymers where some of the acid groups have been neutralized with either zinc or sodium ions (commonly known as ionomers); polyacetals; chloropolymers such as polyvinylchloride (PVC); polyesters such as polyethyleneterephthalate (PET); polyester-ethers; polyamides such as nylon 6 and nylon 6,6; polyamide ethers; polyethers; elastomers such as elastomeric polyurethanes and polyurethane copolymers; silicones; polycarbonates; and mixtures and block or random copolymers of any of the foregoing.

The surface of walls **15** may be coated with a polymer. Walls **15** are illustrated as having a polymer coating on both its outer surface **19** and its inner surface **101.** Magnetically reactive object **13** can also have a polymer coating on its outer surface **102.** The polymer coating on outer surface **19, 102** and inner surface **101** can be a biocompatible polymer, including but not limited to PTFE. Polymer coating can also comprise a hydrophilic polymer selected from the group comprising polyacrylate, copolymers comprising acrylic acid, polymethacrylate, polyacrylamide, poly(vinyl alcohol), poly(ethylene oxide), poly(ethylene imine), carboxymethylcellulose, methylcellulose, poly(acrylamide sulphonic acid), polyactylonitrile, poly(vinyl pyrrolidone), agar, dextran, dextrin, carrageenan, xanthan, and guar. The hydrophilic polymers can also include ionizable groups such as acid groups, e.g., carboxylic, sulphonic or nitric groups. The hydrophilic polymers may be cross-linked through a suitable cross-binding compound. The cross-binder actually-used depends on the polymer system: if the polymer system is polymerized as a free radical polymerization, a preferred cross-binder comprises two or three unsaturated double bonds.

The polymer coating on inner surface **101** and outer surface **19, 102** can also be loaded with a variety of bioactive agents. The bioactive agent preferably includes one or more antimicrobial agents. The term "antimicrobial agent" refers to a bioactive agent effective in the inhibition of, prevention of or protection against microorganisms such as bacteria, microbes, fungi, viruses, spores, yeasts, molds and others generally associated with infections such as those contracted from the use of the medical articles described herein. The antimicrobial agents include antibiotic agents and antifungal agents.

Antibiotic agents may include cephalosporins, clindamycin, chloramphenicol, carbapenems, penicillins, monobactams, quinolones, tetracycline, macrolides, sulfa antibiotics, trimethoprim, fusidic acid and aminoglycosides. Antifungal agents may include amphotericin B, azoles, flucytosine, cilofungin and nikkomycin Z Specific non-limiting examples of suitable antibiotic agents include: ciprofloxacin, doxycycline, amoxicillin, metronidazole, norfloxacin (optionally in cambinaton with ursodeoxycholic acid), ciftazidime, and cefoxitin. Other suitable antibiotic agents include rifampin, minocycline, novobiocin and combinations thereof discussed in U.S. Patent No 5,217,493 (Raad et al.).

Rifampin is a semisynthetic derivative of rifamycin B, a macrocyclic antibiotic compound produced by the mold *Streptomyces mediterranic.* Rifampin is believed to inhibit bacterial DNA-dependent RNA polymerase activity and is bactericidal in mature. Rifampin is available in the United States from Merrill Dow Pharmaceuticals, Cincinnati, Ohio. Minocycline is a semisynthetic antibiotic derived from tetracycline. It is primarily bacteriostatic and is believed to exert an antimicrobial effect by inhibiting protein synthesis. Minocycline is commercially available as the hydrochloride salt which occurs as a yellow, crystalline powder and is soluble in water and slightly soluble in alcohol. Minocycline is available from Lederle Laboratories Division, American Cyanamid Company, Pearl River, N.Y. Novobiocin is an antibiotic obtained from cultures of *Streptomyces niveus* or S *spheroides.* Novobiocin is usually bacteriostatic in action and is believed to interfere with bacterial cell wall synthesis and inhibit bacterial protein and nucleic acid synthesis. Novobiocin also appears to affect stability of the cell membrane by complexing with magneasium. Novobiocin is available from The Upjohn Company, Kalamazoo, Michigan.

The polymer coating is preferably capable of releasing the bioactive agent into the body at a predetermined time and at a predetermined rate Such polymeric coatings include drug-eluting matrix materials described in U.S. Patent Nos. 5,380,299, 6,530,951, 6,774,278 and U S. Patent Application Serial Nos. 10/218,305, 10/223,415, 10/410,587, 10/000,659, and 10/618,977.

Alternatively, different polymer coatmgs can be coated on outer surface **19, 102** and inner surface **101.** For example, the polymer coating on outer surface **19** can include any polymer coating commonly known to those skilled in the art to help reduce tissue irritation incurred as a result of stent **10a** being in contact with a passageway of the patient for a prolonged period of time. The polymer coating on inner surface **101** and outer surface **102** can also include any coating commonly known to those skilled in the art to further help prevent clogging of stent **10a.**

Alternatively, inner surface **101** and outer surface **19, 102** of stent **10a** can be composed from a biodegradable polymer that gradually bioerodes with time. Biodegradable polymers may include rigid dissolvable polymers such as poly(lactid acid), poly(glycolic acid), and poly-epsilon-capro-lactone, or combinations thereof. Other rigid dissolvable polymers will be apparent to those of ordinary skill in the art. Suitable biodegradable polymers may be selected from the group consisting of: a hydrogel, an elastin-like peptide, a polyhydroxyalkanoates (PHA), polyhydroxybutyrate compounds, and co-polymers and mixtures thereof. The biodegradable material can be selected and varied based on various design criteria. The biodegradable material preferably comprises one or more hydrolyzable chemical bonds, such as an ester, a desired degree of crosslinking, a degradation mechanism with minimal heterogeneous degradation, and nontoxic monomers. The biodegradable material is preferably a polyhydroxyalkanoate compound, a hydrogel, poly(glycerol-sebacate) or an elastin-like peptide. Desirably, the biodegradable material comprises a poly-α-hydroxy acid, such as polylactic acid (PLA). PLA can be a mixture of enantiomers typically referred to as poly-D,L-lactic acid. Alternatively, the biodegradable material is poly-L(+)-lactic acid (PLLA) or poly-D(-)-lactic acid (PDLA), which differ from each other in their rate of biodegradation. PLLA is semicrystalline. In contrast, PDLA is amorphous, which can promote the homogeneous dispersion of an active species. Unless otherwise specified, recitation of "PLA" herein refers to a biodegradable polymer selected from the group consisting of: PLA, PLLA and PDLA.

Stent **10a** may also comprise a drug-releasing which may be formed by any suitable process conventionally used to shape polymeric materials such as thermoplastic and elastomeric materials. Shaping processes can include, but not limited to, extrusion including coextrusion, molding, calendaring, casting and solvent coating. Preferred shaping processes include extrusion and coextrusion processes. For example, a biodegradable coating polymer mixed with a drug may be applied to inner surface **101** of stent **10a** by applying a solvent solution or liquid dispersion of a biodegradable polymer onto a surface of walls **15** followed by removing the solvent or liquid dispersing agent, *e.g*., by evaporation. Such a solution or dispersion of the biodegradable polymer may be applied by contacting a surface of the support member with the solution or dispersion by, for example, dipping or spraying. For example, the biodegradable coating may be applied by spraying a solution of a biodegradable polymer onto walls **15** within lumen **14** of stent **10a.** Alternatively, a coated stent **10a** can be formed by applying a polymer to the exterior surface of a biodegradable coating to form a multilayer medical device. For example, a solution of a biostable polymer can be applied to the external surface of a tube of the biodegradable coating and dried in place to form stent **10a.**

Alternatively, each of the multiple layers may be solvent cast. The second layer is cast from a solvent that does not dissolve the already-cast layer. For example, a polyurethane used to form stent **10a** may be dissolved in dimethylformamide, while PLA used to form a biodegradable coating may be dissolved in dichloromethane. Where the second solvent does not dissolve the support member polymer, the second solution may be spread on the first layer once dry, and the solvent evaporated off. The resulting multi-layers have a strong bond between the layers.

Biodeposition-reducing bioactive agents can be selected to withstand the extrusion temperature. In a first aspect, a bioactive agent may be included within, or mixed with, the polymer prior to extrusion. Extrusion of the film allows inclusion of a drug or agent that can withstand the extrusion temperatures. For example, the antimicrobial agents described in U.S. patent application US2005/0008763A1 are compatible with this manufacturing technique.

The bioactive agent preferably does not materially interfere with the physical or chemical properties of the biodegradable material in which it is included. The bioactive agent and the biodegradable material may be preformed using any of the conventional devices known in the art for such purposes. Where thermoplastic materials are employed, a polymer melt may be formed by heating the various agents, which can then be mixed to form a homogenous mixture. A common way of doing so is to apply mechanical shear to a mixture of the matrix polymer and additives. Devices in which the biodegradable material and the bioactive(s) may be mixed in this fashion include, but are not limited to, devices such as a single screw extruder, a twin screw extruder, a banbury mixer, a high-speed mixer, and a ross kettle.

In a second aspect, the biodegradable coating is adhered to stent **10a** without a bioactive agent, and the bioactive agent may be subsequently absorbed into the biodegradable coating after the formation of the device. For example, the biodegradable coating can be contacted with a solution of the bioactive agent within the drainage lumen **16** of stent **10a.** The effective concentration of the bioactive agent within the solution can range from about 1 to 10 µg/ml for minocycline, preferably about 2 µg/ml; 1 to 10 µg/ml for rifampin, preferably about 2 µg/ml; and 1 to 10 µg/ml for novobiocin, preferably about 2 µg/ml. The solution is preferably composed of sterile water or sterile normal saline solutions.

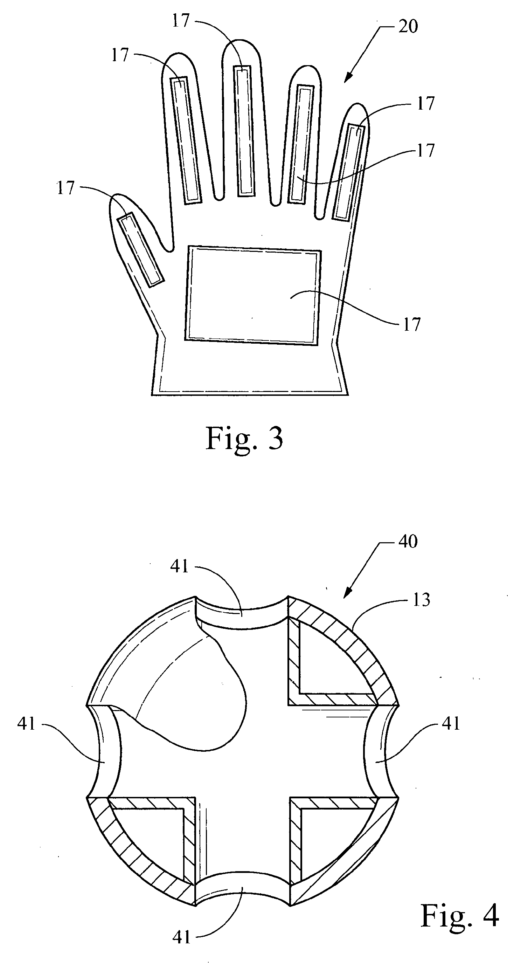

**Fig. 3** depicts an alternate actuation device **20** having strips of magnets **17** located on the fingers and the palm. Magnetic strips are flexible so that one using the glove is able to massage the area above stent **10a** and actuate magnetically reactive objects by manually manipulating magnetically reactive object therein; however, it is not required that magnets be flexible. Like glove **10b,** glove **20** is not limited to having two or more magnets nor a magnet on each finger or palm.

**Figs. 4****,** **5,** and **6** depict alternative magnetically reactive objects **40, 50, 60** for use in a stent such as that depicted in, but not limited to, **Figs. 1A****,** **7,** and **8****.** Magnetically reactive object **40** has a plurality of lumens **41** to permit fluid flow therethrough. It is contemplated that magnetically reactive object **40** may have more or less lumens than that depicted in **Fig. 4****.** Because lumens **41** permit fluid flow therethrough, magnetically reactive object **40** does not restrict as much fluid flow relative to a magnetically reactive object having a continuous surface.

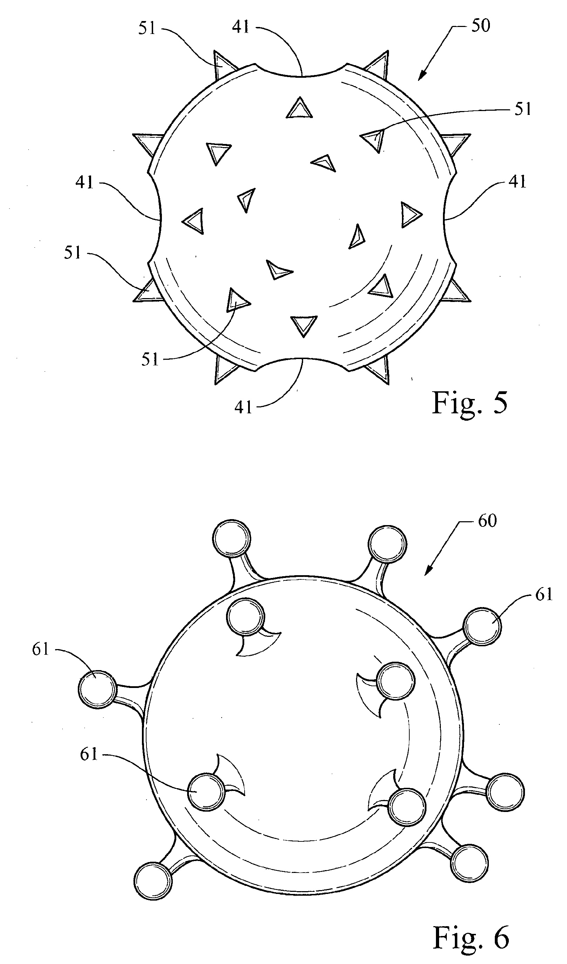

Magnetically reactive object may also include projections to provide additional contact with stent wall. For example, magnetically reactive object **50** depicted in **Fig. 5** includes one or more bristles **51** to aid in the removal of cumulative matter when bristle **51** contacts wall **15.** Other types of projections are also contemplated, including but not limited to points, bumps, and other protrusions. Likewise, magnetically reactive object **60** depicted in **Fig**. **6** (having a shape similar to a jack) has one or more nob protrusions **61** to aid in the removal of cumulative matter when nob **61** contacts wall **15.** Each of the magnetically reactive objects described and contemplated herein are not limited to the specific shape depicted in the figures, nor are they limited to a specific number of lumens; each may have any number of lumens-including none.

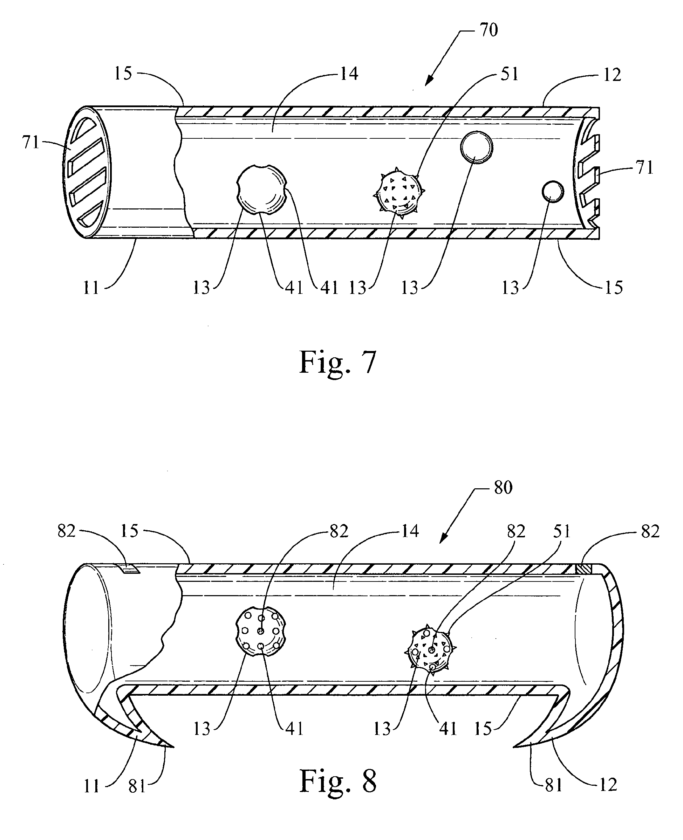

**Fig. 7** depicts an alternate stent **70** having a plurality of magnetically reactive objects **13** having different characteristics and sizes and end caps **71** to retain one or more magnetically reactive objects **13** within lumen **14.** End cap securing mechanisms **71** are extruded as part of walls 15, although it is contemplated that end caps **71** could be separate pieces fixedly attached to first portion **11** and second portion **12** of stent **70.** Additionally, it is contemplated that end caps **71** could reside within walls **15.** End caps **71** prevent the escape of magnetically reactive object **13** from lumen **14** and have openings to allow fluid to pass therethrough. End cap **71** is not limited to having a slotted-shape, but may include other configurations that are able to prevent the escape of magnetically reactive object **13** from lumen **14** and allow fluid to pass therethrough, including but not limited to, a cross-shape.

**Fig. 8** depicts an alternate stent **80** having securing mechanisms barbs **81** that are tapered to retain magnetically reactive object **13** within lumen **14** and stent **80** within a bodily cavity. Stent **80** may also include radiopaque marker **82,** radiopaque filler, or may be made from a radiopaque material such that stent **80** can be viewed using an imaging device, including but not limited to, a fluoroscope, x-ray, and ultrasound. The imaging device permits medical personnel to view stent **80** disposed within a patient. Additionally, magnetically reactive object **13** may have a radiopaque marker **82,** radiopaque filler, or may be made from a radiopaque material such that it is observable using an imaging device.

Using imaging device, medical personnel are able to observe the actuation of magnetically reactive object **13** relative to stent **80** to determine if actuation of magnetically reactive object **13** is sufficient to restore the patency of the stent. For example, if magnetically reactive object **13** is unable to move about within stent **80,** it may mean that stent **80** is sufficient clogged so as to prevent the movement of magnetically reactive object **13.** Accordingly, the medical professional may continue actuating magnetically reactive object **13** until a sufficient flow-path is created. Alternatively, the medical professional can use a different actuation device having different properties, including but not limited to, a stronger or weaker magnet, to create a stronger or weaker reaction between the actuation device and magnetically reactive object **13.** Once actuation device is able to move magnetically reactive object **13** sufficiently about stent **80,** it can be believed that stent **80** is sufficiently free from cumulative master so as to not significantly occlude stent **80**. Additionally, by using actuation devices having different properties, including but not limited to, a stronger magnet or weaker magnet, the medical professional is able to actuate magnetically reactive object **13** amongst different planes and accordingly have magnetically reactive object **13** contact the near and far walls **15** of stent **80.**

The foregoing description and drawings are provided for illustrative purposes only and are not intended to limit the scope of the invention described herein or with regard to the details of its construction and manner of operation. It will be evident to one skilled in the art that modifications and variations may be made without departing from the scope of the invention. Changes in form and in the proportion of parts; as well as the substitution of equivalence, are contemplated as circumstances may suggest and render expedience; although specific terms have been employed, they are intended in a generic and descriptive sense only and not for the purpose of limiting the scope of the invention set forth in the following claims.

## Claims

1. A system for maintaining the patency of a plastic tubular stent through actuated movement to dislodge cumulative matter from the stent, wherein the system comprises:
a plastic tubular stent (10a) having a lumen (14);
an object (13) being freely moveable within the lumen and configured to at least partially dislodge cumulative matter deposited within the plastic tubular stent; and
first and second securing mechanisms configured to retain the object within the lumen, wherein each securing mechanism comprises a tapered portion (11, 12) of the stent; an end cap (71) or a tapered barb (81);
**characterised in that**:
the system further comprises an actuation device (10b) separate from the plastic tubular stent; and
the object (13) of the plastic tubular stent consists of a magnetically reactive object configured to at least partially dislodge cumulative matter, deposited within the plastic tubular stent, when the actuation device is passed near the plastic tubular stent.

2. The system of Claim 1 wherein the actuation device is selected from the group consisting of a device configured to be temporarily attached to a hand, a device configured to be at least partially disposed about the hand, a cloth member, a mitt, a mitten, a glove, a wand, a strap, a housing, and a magnet.

3. The system of Claim 1 wherein the system further comprises an imaging device for viewing at least a portion of the plastic tubular stent or one or more magnetically reactive objects disposed therein.

4. The system of Claim 3 wherein the imaging device is selected from the group consisting of a fluoroscope, ultrasound, and x-ray.

5. The system of Claim 1 wherein the magnetically reactive object of the plastic tubular stent further comprises two or more magnetically reactive objects.

6. The system of Claim 1 wherein the magnetically reactive object of the plastic tubular stent is a rare earth magnet.

7. The system of Claim 1 wherein the magnetically reactive object of the plastic tubular stent further comprises a plurality of lumens configured to permit the passage of fluid therethrough.

8. The system of Claim 1 wherein the plastic tubular stent or the magnetically reactive object further comprises a radiopaque marker or radiopaque filler.

9. The system of Claim 1 wherein the magnetically reactive object of the plastic tubular stent has one or more projections disposed about the surface.

10. The system of Claim 1 wherein at least one of an inner surface of the plastic tubular stent, an outer surface of the plastic tubular stent, and the magnetically reactive object of the plastic tubular stent is coated with a polymer.

11. The system of Claim 1 wherein the magnetically reactive object of the plastic tubular stent or the actuation device further comprises a rare earth magnet.

## Patentansprüche

1. System zur Aufrechterhaltung der Durchgängigkeit eines röhrenförmigen Stents aus Kunststoff durch betätigte Bewegung, um angesammeltes Material von dem Stent zu lösen, worin das System Folgendes umfasst:
einen röhrenförmigen Stent (10a) aus Kunststoff mit einem Lumen (14);
einen Gegenstand (13), der frei in dem Lumen beweglich ist und ausgelegt ist, angesammeltes Material, das sich auf dem röhrenförmigen Stent aus Kunststoff abgelagert hat, zumindest teilweise zu entfernen; und
erste und zweite Befestigungsmechanismen, die ausgelegt sind, den Gegenstand in dem Lumen zu halten, worin jeder Befestigungsmechanismus einen verjüngten Abschnitt (11, 12) des Stents umfasst;
eine Endkappe (71) oder einen verjüngten Widerhaken (81);
**dadurch gekennzeichnet, dass**:
das System ferner eine von dem röhrenförmigen Stent aus Kunststoff separate Betätigungsvorrichung (10b) umfasst; und der Gegenstand (13) des röhrenförmigen Stents aus Kunststoff aus einem magnetisch reaktionsfähigen Gegenstand besteht, der ausgelegt ist, angesammeltes Material, das sich in dem röhrenförmigen Stent aus Kunststoff abgelagert hat, zumindest teilweise zu lösen, wenn die Betätigungsvorrichtung in der Nähe des röhrenförmigen Stents aus Kunststoff vorbeigeführt wird.

2. System nach Anspruch 1, worin die Betätigungsvorrichtung aus der aus einer Vorrichtung zur temporären Befestigung an einer Hand, einer Vorrichtung zur mindestens teilweisen Anordnung um die Hand, einem Tuchelement, einem Fausthandschuh, einem fingerlosen Handschuh, einem Handschuh, einem Stab, einem Gurt, einem Gehäuse und einem Magneten bestehenden Gruppe ausgewählt ist.

3. System nach Anspruch 1, worin das System ferner eine bildgebende Vorrichtung zur Betrachtung zumindest eines Teils des röhrenförmigen Stents aus Kunststoff oder von einem oder mehreren darin angeordneten magnetisch reaktionsfähigen Gegenständen umfasst.

4. System nach Anspruch 3, worin die bildgebende Vorrichtung aus der aus einem Fluoroskop, Ultraschall und Röntgen bestehenden Gruppe ausgewählt ist.

5. System nach Anspruch 1, worin der magnetisch reaktionsfähige Gegenstand des röhrenförmigen Stents aus Kunststoff zwei oder mehr magnetisch reaktionsfähige Gegenstände umfasst.

6. System nach Anspruch 1, worin der magnetisch reaktionsfähige Gegenstand des röhrenförmigen Stents aus Kunststoff ein Magnet aus seltenen Erden ist.

7. System nach Anspruch 1, worin der magnetisch reaktionsfähige Gegenstand des röhrenförmigen Stents aus Kunststoff ferner eine Vielzahl von Lumen umfasst, die ausgelegt sind, die Durchleitung von Flüssigkeit durch sie hindurch zu gestatten.

8. System nach Anspruch 1, worin der röhrenförmige Stent aus Kunststoff oder der magnetisch reaktionsfähige Gegenstand ferner einen röntgenopaken Marker oder einen röntgenopaken Füllstoff umfasst.

9. System nach Anspruch 1, worin der magnetisch reaktionsfähige Gegenstand des röhrenförmigen Stents aus Kunststoff einen oder mehrere Vorsprünge aufweist, die um die Oberfläche angeordnet sind.

10. System nach Anspruch 1, worin zumindest eine einer Innenfläche des röhrenförmigen Stents aus Kunststoff, einer Außenfläche des röhrenförmigen Stents aus Kunststoff und des magnetisch reaktionsfähigen Gegenstands des röhrenförmigen Stents aus Kunststoff mit einem Polymer beschichtet ist.

11. System nach Anspruch 1, worin der magnetisch reaktionsfähige Gegenstand des röhrenförmigen Stents aus Kunststoff oder die Betätigungsvorrichtung ferner einen Magneten aus seltenen Erden umfassen.

## Revendications

1. Système de maintien de la perméabilité d'un stent tubulaire en plastique par le biais d'un mouvement commandé pour déloger des substances accumulées dans le stent, le système comprenant :
un stent tubulaire en plastique (10a) ayant une lumière (14) ;
un objet (13) pouvant se déplacer librement à l'intérieur de la lumière et configuré pour déloger au moins en partie des substances accumulées déposées à l'intérieur du stent tubulaire en plastique ; et
des premier et deuxième mécanismes de fixation configurés pour retenir l'objet à l'intérieur de la lumière, chaque mécanisme de fixation comprenant une portion effilée (11, 12) du stent ;
un capuchon d'extrémité (71) ou une barbe effilée (81) ;
**caractérisé en ce que** :
le système comprend en outre un dispositif d'activation (10b) séparé du stent tubulaire en plastique ; et
l'objet (13) du stent tubulaire en plastique est constitué d'un objet magnétiquement réactif configuré pour déloger au moins en partie des substances accumulées, déposées à l'intérieur du stent tubulaire en plastique, lorsque le dispositif d'activation passe à proximité du stent tubulaire en plastique.

2. Système selon la revendication 1, dans lequel le dispositif d'activation est sélectionné parmi le groupe constitué d'un dispositif configuré de manière à être attaché temporairement à une main, un dispositif configuré pour être au moins en partie disposé autour de la main, un organe de type étoffe, une manique, une moufle, un gant, une baguette, une languette, un boîtier, et un aimant.

3. Système selon la revendication 1, dans lequel le système comprend en outre un dispositif d'imagerie pour visualiser au moins une portion du stent tubulaire en plastique ou un ou plusieurs objets magnétiquement réactifs disposés dans celui-ci.

4. Système selon la revendication 3, dans lequel le dispositif d'imagerie est sélectionné parmi le groupe constitué d'un fluoroscope, d'un dispositif à ultrasons, et d'un dispositif de radiographie.

5. Système selon la revendication 1, dans lequel l'objet magnétiquement réactif du stent tubulaire en plastique comprend en outre deux ou plus de deux objets magnétiquement réactifs.

6. Système selon la revendication 1, dans lequel l'objet magnétiquement réactif du stent tubulaire en plastique est un aimant aux terres rares.

7. Système selon la revendication 1, dans lequel l'objet magnétiquement réactif du stent tubulaire en plastique comprend en outre une pluralité de lumières configurées pour permettre le passage de fluide à travers elles.

8. Système selon la revendication 1, dans lequel le stent tubulaire en plastique ou l'objet magnétiquement réactif comprend en outre un marqueur radio-opaque ou une charge radio-opaque.

9. Système selon la revendication 1, dans lequel l'objet magnétiquement réactif du stent tubulaire en plastique présente une ou plusieurs saillies disposées autour de la surface.

10. Système selon la revendication 1, dans lequel au moins l'un(e) d'une surface interne du stent tubulaire en plastique, d'une surface externe du stent tubulaire en plastique, et de l'objet magnétiquement réactif du stent tubulaire en plastique est revêtu(e) d'un polymère.

11. Système selon la revendication 1, dans lequel l'objet magnétiquement réactif du stent tubulaire en plastique ou le dispositif d'activation comprend en outre un aimant aux terres rares.
